(19) Europäisches Patentamt

European Patent Office

Office européen des brevets

(11) **EP 0 553 085 B1**

(12) **EUROPEAN PATENT SPECIFICATION**

(45) Date of publication and mention of the grant of the patent:
**20.10.1999 Bulletin 1999/42**

(21) Application number: **91910886.0**

(22) Date of filing: **14.05.1991**

(51) Int Cl.6: **C12P 23/00**, C12N 1/12, C12N 1/18, C12N 1/16, C12N 1/14, C12N 1/20

(86) International application number:
**PCT/US91/03358**

(87) International publication number:
**WO 91/18108 (28.11.1991 Gazette 1991/27)**

(54) **A HIGH PRODUCTIVITY, CONTINUOUS FERMENTATION PROCESS FOR CAROTENOID-PRODUCING MICROORGANISMS**

EIN KONTINUIERLICHER FERMENTATIONSPROZESS ZUR HERSTELLUNG VON MIKROORGANISMEN MIT EINER HOHEN PRODUKTIVITÄT AN CAROTENOIDEN

PROCEDE DE FERMENTATION CONTINUE DE HAUT RENDEMENT POUR DES MICROORGANISMES PRODUISANT DES CAROTENOIDES

(84) Designated Contracting States:
**DE ES FR GB GR IT NL**

(30) Priority: **15.05.1990 US 524140**

(43) Date of publication of application:
**04.08.1993 Bulletin 1993/31**

(73) Proprietor: **ARCHER DANIELS MIDLAND COMPANY Decatur, Illinois 62526 (US)**

(72) Inventors:
 • **BAILEY, Richard, B. Fort Collins, CO 80526 (US)**
 • **MEDWID, Richard, D. Fort Collins, CO 80526 (US)**

(74) Representative: **Griffin, Kenneth David et al Saunders & Dolleymore, 9, Rickmansworth Road Watford, Hertfordshire WD1 7HE (GB)**

(56) References cited:
   CA-A- 729 603          US-A- 3 108 402

   • **Proceedings International Fermentation Symposium, 4th: Fermentation Technology, issued 1972 (Japan), ENDO et al., "Studies on the heterotrophic growth of Chlorella in a Mass culture", pages 433-541, see entire document.**
   • **STANBURY et al., "Principles of fermentation Technology", published 1984, pages 14-18, see entire document.**
   • **Journal of Phycology, Volume 13, issued 1977, DEASON et al., "Taxonomic significance of secondary carotenoid formation in Neospongiococcum (chlorococcales, chlorophyta)", pages 176-180, see entire document.**

**Description**

The present invention is directed to a method for the efficient fermentation of heterotrophic algae which produce carotenoids and, in particular, a continuous process for fermenting Neospongiococcum algae in a manner designed to maximize the level of xanthophyll in the algae.

Batch and continuous fermentation methods are known. In certain batch fermentation processes, the microorganism populations increase to a certain level and then the growth rate decreases or ceases altogether. In particular, this problem has been discovered in connection with the unicellular green algae Neospongiococcum excentricum. This alga is useful due to its production of carotenoids.

Deason in J. Phycol, 13, 176-180, (1977) described secondary carotenoid formation in some species of Neospongiococcum.

Carotenoids are accessory pigments in photosynthetic microorganisms which function by absorbing light and transferring energy to chlorophyll for photosynthesis. Carotenoids also act to protect cells from photooxidative damage from oxygen radicals produced during photosynthesis. See generally Spurgeon, et al., The Biochemistry of Plants, Chap. 14 (1980).

Although the occurrence of carotenoids among photosynthetic organisms is ubiquitous, the occurrence among dark green heterotrophic algae is somewhat less common. Carotenoids, and xanthophylls in particular, have been observed to be formed under heterotrophic conditions by certain bacteria, yeasts and unicellular algae, with the highest levels observed in the latter.

Xanthophylls are oxygen-containing carotenoids and are useful as natural coloring agents. Xanthophylls and, in particular, lutein and zeaxanthin, are used as feed supplements for poultry to produce a yellowish color in skin, shanks and egg yolks.

Production of carotenoids by fermentation of microorganisms is known. In Farrow, U.S. Patent No. 3,280,502 (1966), a process for the preparation of lutein is disclosed in which a strain of Chlorella pyrenoidosa is fermented and up to 235 mg of lutein per liter is produced in ninety-six hours. Kathrien, U.S. Patent No. 2, 949, 700 (1960) discloses a process for the production of carotenoids by the cultivation of algae of the phyla Chlorophyta, including genera of Chlorococcum, Chlorella, and Chlamydomonas. Microbiological production of carotenoids by fungi is also known. For example, see Farrow, U.S. Patent No. 2,974,044 (1961). However, a problem with prior art processes is that the amount of carotenoid produced is typically too low to be economically feasible.

U.S. Patent No. 3,108,402 describes a method of producing carotenoids such as beta-carotene and xanthophyll by the cultivation of algae of the division Chlorophyta and of the genus Chloretta, Chlorococcum, Spongiochloris, Spongiococcum and Coccomyxa.

The unicellular green alga Neospongiococcum excentricum produces xanthophylls and has been used as a feed additive to provide color. See, for example, Marusich, et al., Oxycarotenoids in Poultry Pigmentation, Poultry Sci. 49 (6) pp. 1555-1566 (1970).

While Neospongiococcum excentricum are known for producing carotenoids, when batch fermentation processes are employed to produce the algae, a barrier to growth and productivity has been discovered. In particular, the growth rate of the population is maintained for approximately 90 hours, after which time productivity declines and growth eventually ceases. Accordingly, it would be advantageous to overcome this barrier to growth and productivity encountered during the fermentation of Neospongiococcum excentricum. Additionally, it would be advantageous to develop fermentation methods in which algae is produced having high levels of carotenoids.

Summary of the Invention

In accordance with the present invention, a continuous fermentation process has been developed which results in a high level of growth and productivity that is sustainable over an extended period of time. As used herein, the term "continuous fermentation process" means a fermentation process in which fresh fermentation medium is added and spent fermentation broth is removed, either without interruption or on a periodic basis. Preferably, the fresh fermentation medium is continuously added to a fermentation vessel and spent fermentation broth is continuously removed from the vessel in order to keep the volume of fermentation broth in the vessel substantially constant over a period of time. As used herein, the term "fresh fermentation broth" refers to that fermentation medium which is added to the fermentation vessel, the term "fermentation broth" refers to the fermentation culture (e.g. medium, cells and fermentation products) contained within the fermentation vessel and the term "spent fermentation broth" refers to the fermentation culture removed from the fermentation vessel.

In accordance with an embodiment of the present invention, a continuous fermentation process is provided in which a Neospongiococcum algae culture is efficiently grown and a high level of carotenoids are produced. The high level of productivity is sustained over a long period of time by dilution of the fermentation broth with fresh fermentation medium. Additionally, the amount of the carbon source added to the fermentation broth is controlled. The carbon source, for example glucose, is fed to the microorganisms at a rate designed to maximize the production of carotenoids, and in particular xanthophyll, without unduly limiting the length of time during which successful fermentation can be accomplished. Temperature and pH are also controlled.

In accordance with another embodiment of the present invention, the growth of <u>Neospongiococcum</u> algae is maximized and the production of xanthophyll is maximized by continuously diluting the fermentation broth with fresh fermentation medium, by limiting the carbon source which is available to the algae, by maintaining the temperature of the fermentation broth between 30°C and 38°C and by controlling the pH of the fermentation broth between about pH 5.5 and about pH 6.5. Preferably, the productivity of xanthophyll is at least 10 mg xanthophyll per liter of fermentation broth per hour of fermentation time, more preferably 15 mg/l/hr and most preferably 20 mg/1/hr.

## Brief Description of the Drawings

Figure 1 is a graph illustrating the effect of temperature on growth rate.

Figure 2 is a graph illustrating the effect of temperature on the percent carotenoid content of the cells.

Figure 3 is a graph illustrating the effect temperature changes have on the percent carotenoid content.

Figure 4 is a graph illustrating the effect temperature changes have on the dry cell weight and the percent carotenoid content.

Figure 5 is a graph illustrating the effect variations in pH have on the growth rate.

Figure 6 is a graph illustrating the effect variations in pH have on the percent carotenoid content.

Figure 7 is a graph illustrating the changes in dry cell weight, percent carotenoid content and productivity for a fermentation period of 90 hours, when cell growth ceases.

## Detailed Description of the Invention

The present invention involves a method for maintaining continuous fermentation of microorganisms in a fermentation broth, and, in particular, for continuously fermenting heterotrophic microorganisms which produce carotenoids. The method includes diluting the fermentation broth with an effective amount of fresh fermentation medium which contains a growth rate-limiting amount of carbon source.

By practicing the present invention, the productive phase of a fermentation can be effectively extended. As a result, depending upon the nature of the fermentation, high cell densities can be achieved and/or the production of materials, such as pigmentation compounds, can be increased. The method further includes controlling various fermentation parameters, including temperature and pH, to improve cell growth and pigmentation content in heterotrophic algae.

This method is particularly advantageous for a strain of <u>Neospongiococcum</u> <u>excentricum</u> identified by ATCC No. 40335, this strain having been deposited with the ATCC under the terms of the Budapest Treaty.

The present invention involves conducting fermentation while diluting the fermentation broth by the addition of fresh fermentation medium. The fresh fermentation medium is generally an aqueous solution added to a fermentation vessel which includes a growth rate limiting amount of a carbon source. In particular, the fresh fermentation medium can be water, or water with nutrients, such as glucose, and other materials added to it.

The fermentation broth can be diluted either by increasing the overall volume with the addition of fresh fermentation medium or, preferably, by maintaining a substantially constant volume of fermentation broth by the addition of fresh fermentation medium and the removal of spent fermentation broth. In the latter case, the dilution rate (D) is equal to the volumetric flow rate of fresh fermentation medium into the fermentation vessel and spent fermentation broth out of the vessel divided by volume of the fermentation broth. The units for D are reciprocal time, and for present purposes will be $hr^{-1}$ unless indicated otherwise. Dilution is equal to the number of vessel liquid volumes which pass through the vessel per hour, and is the reciprocal of mean residence time.

The addition of fresh fermentation medium and the removal of spent fermentation broth can be either constant or periodic, but is preferably constant. The addition of fresh fermentation medium and the removal of spent fermentation broth can either be simultaneous or not, but is preferably simultaneous.

The present method requires a dilution rate which is less than the maximum growth rate ($u^{max}$). As used herein, the term "growth rate" (which will be indicated by the symbol "u") is the mass of cells formed per mass of cells in the fermentation broth per unit time. Unless indicated otherwise, the units for growth rate will be $hr^{-1}$, and the symbol will be "$u^{hr-1}$".

In the case of maintaining a relatively constant volume of fermentation broth for the growth of a typical culture, dilution is typically effective at between about $0.03 \ hr^{-1}$ to about $0.13 \ hr^{-1}$, more preferably between about $0.06 \ hr^{-1}$ to about $0.12 \ h^{-1}$ and most preferably between about $0.09 \ hr^{-1}$ and about $0.11 \ hr^{-1}$.

At steady state, D = u in the continuous fermentation method of the present invention. Dilution rate (D) is dictated by the maximum growth rate ($u^{max}$) of a particular organism. This can be determined by measuring $u^{max}$ in a batch culture using a nutrient-rich medium (e.g one having an excess of required nutrients) under optimal conditions. One may set the dilution rate in continuous culture at any level less than $u^{max}$. For optimal productivity, D is preferably from about 25% to about 95% of $u^{max}$, and more preferably is about 90% of $u^{max}$. The system will tend to be more unstable as $u^{max}$ is approached. Washout occurs if D is greater than u.

It should be recognized that with the introduction of fresh fermentation medium, the nutrient levels in the fermentation broth must be maintained at concentrations sufficient to support growth and/or production of extracellular material. Such nutrients can be added either in the fresh fermentation medium or independently of it. Specifically, such nutrients include sources of carbon; nitrogen; phosphates; sulfates; and magnesium, iron, and other trace metals.

Another means of increasing productivity is by increasing the concentration of the carbon source in the feed which will increase the biomass concentration. However, an embodiment of the present invention includes conducting a fermentation while restricting the carbon source in the fermentation to a growth rate-limiting level. In this manner, it has been discovered that the time of fermentation can be extended indefinitely. In addition, by selectively limiting the carbon source in the fermentation, the yield factor ($Y_{x/s}$) can be increased. As used herein, the term "yield factor" ($Y_{x/s}$) refers to the amount of cell mass (X) formed for every mass unit of substrate (S) consumed. In the present instance, the substrate is the carbon source.

The foregoing aspect of the present invention involves restricting the rate of addition of the carbon source to a rate-limiting level. Preferably, all other nutrients necessary for growth are present in excess compared with the carbon source. In other words, if the rate of addition of the carbon source were to be increased, the growth rate would increase. However, if the rate of addition of the other nutrients were to be increased, the growth rate would remain substantially unchanged. Without intending to be bound by theory, it is believed that by restricting the availability of the carbon source, the production of toxins is prevented or substantially reduced because microorganisms will be forced to preferentially utilize available carbon for growth.

The rate of addition of the carbon source can be monitored and the carbon source can be fed at a rate to achieve the desired restricted growth rate. The carbon source is fed on a continuous basis to maintain the desired restricted level. Specifically, it has been determined that it is useful to maintain the feed of the carbon source at a rate designed to restrict the growth rate to a range from about 25% of $u^{max}$ to about 95% of $u^{max}$, more preferably to about 90% of $u^{max}$.

The embodiment of the present invention relating to carbon source restriction is effective for use with any carbon source used in a fermentation. Specifically, the invention is useful for any type of carbohydrate and, in particular, any monosaccharide, disaccharide or trisaccharide. In particular, for the fermentation of <u>Neospongiococcum,</u> the carbon source is preferably glucose.

In the practice of the present method of restricting the feed rate of a carbon source to obtain the benefits of extended fermentation and improved product yield, it is recognized that by restricting the carbon source addition rate too greatly, the ability to grow and/or produce materials may be negatively affected to an undesirable extent. Therefore, it is desirable to maintain the carbon source feed rate at the desired restricted level but to also maintain a sufficient carbon source feed rate to avoid having a negative impact on growth and/or production of extracellular materials.

In a preferred embodiment of the present invention the fermentation broth is diluted with fresh fermentation medium and the carbon source feed rate is restricted to a rate-limiting level. Preferably, this method includes three phases: (1) an inoculation phase; (2) a fed-batch growth phase; and (3) a carbon source limited, continuous culture growth phase. The inoculation phase includes providing an inoculum of actively growing microorganisms to a fermentation vessel. The fed-batch growth phase includes propagating microorganisms in the fermentation vessel with continuous medium additions while maintaining the carbon source concentration at less than 5 grams per liter. Typically, the carbon source concentration is between about 1 g/l and about 5 g/l during the fed-batch period. During the transition from the fed-batch phase to the steady state continuous phase, the rate of continuous feed of fresh fermentation medium containing the carbon source is gradually increased. The continuous fermentation phase commences when a desired steady state cell concentration is attained.

Preferably, the carbon source is a carbohydrate, such as glucose. Prior to switching to the glucose limited, continuous culture growth phase, glucose is depleted to a minimal concentration. This concentration is as low as practical, typically about 1 g/l. The fermentation is then switched to the glucose limited, continuous culture growth phase. A continuous feed stream, including glucose and otherwise nutritionally balanced for growth (i.e. including nutrients such as potassium, phosphate, iron, sulfate, citric acid, magnesium and other trace metals) is introduced to the fermentation medium at an initially low dilution rate. The dilution rate is then gradually increased until the growth rate of the microorganisms is almost as great as the maximum growth rate achieved during the batch growth phase and a desired steady-state dilution rate (D=u) is attained. Preferably D is about 90% $u^{max}$. Fresh fermentation medium is added at a rate equal to the removal rate for the spent fermentation broth.

In conjunction with the process parameters of the present invention, as described above, the present invention includes the control of additional process parameters. In the fermentation of heterotrophic algae, temperature, pH, dissolved oxygen content and carbon dioxide level are controllable process parameters. The present invention further includes control of these variables to obtain high growth rates and high yields of carotenoids. Preferably, the volumetric productivity of xanthophyll is at least 10 mg xanthophyll per liter of fermentation broth per hour, more preferably 15 mg/1/hr and most preferably 20 mg/1/hr.

The following discussion regarding control of the temperature, pH, dissolved oxygen and carbon dioxide process parameters is generally applicable to all heterotrophic algae, including Neospongiococcum. More particularly, the following discussion is especially applicable to a strain of Neospongiococcum excentricum identified by ATCC No. 40335.

With regard to temperature, it has been found that maximum growth of heterotrophic algae can be achieved over a specific range of temperatures. In particular, high growth rates have been found between about 30°C and about 38°C and more particularly between about 33°C and about 38°C. The growth rate (u) is equal to the slope of a best-fit line drawn through a plot of the points indicating dry cell weight at various time intervals drawn on a semi-logarithmic scale, in other words, the slope of a line representing the plot of the log of the dry cell weight versus time.

It has also been determined that carotenoid levels are maximized by conducting the fermentation at 36°C. It will be recognized that the present invention can be conducted within a range of temperatures around 36°C, for example, the fermentation can be conducted from about 33°C to about 38°C.

It has also been found that control of pH in fermentation of heterotrophic algae is important to maximize growth and carotenoid production. In particular, it has been found that heterotrophic algae have a maximum growth rate between about pH 5.5 and about pH 6.5. Below about pH 5.5 and above about pH 6.5, growth rate drops rapidly. It should be recognized, however, that reference to pH between "about" pH 5.5 and "about" pH 6.5 includes pH values just less than pH 5.5 and just greater than pH 6.5 to the extent such values provide for a growth rate substantially equivalent to growth rates obtained by operating between pH 5.5 and pH 6.5.

It has further been determined that carotenoid production is maximized by conducting fermentation at about pH 6.0. Reference to "about" pH 6.0 includes values which are less than and greater than pH 6.0, but which are close enough to pH 6.0 to obtain high values of carotenoid production.

In view of the foregoing discussion relating to the effect of pH on growth rates of heterotrophic algae and on carotenoid content of heterotrophic algae, the present invention includes conducting fermentations and controlling growth rate and/or carotenoid content by controlling pH in accordance with the above-described parameters. Specifically, growth rate is maximized at between about pH 5.5 and about pH 6.5. Alternatively or in addition, carotenoid content is maximized by controlling pH at about pH 6.0. The effect of pH on carotenoid formation has further been found to be reversible. More particularly, the effect of pH on carotenoid formation was found to be reversible within about three generations. Accordingly, the present invention includes conducting fermentations and adjusting pH to an appropriate level in accordance with the above parameters at a point in the fermentation adequate to obtain desired carotenoid levels at a necessary time. For example, it may be desirable to conduct a fermentation at a pH at which carotenoid formation is not maximized for a portion of the fermentation and subsequently adjusting pH several generations prior to termination of the fermentation to a level at which carotenoid formation is maximized. An analogous situation exists with respect to other parameters, e.g. temperature, dissolved oxygen level and carbon dioxide level, as will be explained in more detail in connection with the Examples set forth hereinafter.

The level of dissolved oxygen in fermentation medium of heterotrophic algae has also been found to be of importance. It has been found that growth and carotenoid formation is relatively constant at dissolved oxygen levels equal to or greater than about 10%. However, it has also been found that at dissolved oxygen levels below about 10%, carotenoid formation can be drastically reduced. Accordingly, another aspect of the present invention includes maintaining the level of dissolved oxygen in fermentations of heterotrophic algae above at least about 10%. Further, it should be noted that appropriate levels of dissolved oxygen depend in large part upon fermentation vessel configuration and mixing patterns. Specifically, at relatively low levels of dissolved oxygen close to a critical level, it is necessary to maintain sufficient mixing to avoid localized areas of fermentation medium having dissolved oxygen content below the minimum necessary to avoid a decrease in growth and carotenoid formation.

It has also been found that carbon dioxide concentration in the fermentation medium is important to carotenoid formation. Specifically, levels of carbon dioxide greater than a partial pressure of about 0.04 are detrimental to carotenoid formation. It has further been found that the effect of carbon dioxide on carotenoid levels is reversible in less than one generation. Accordingly, the present invention includes conducting fermentation and maintaining the concentration of carbon dioxide below a partial pressure of about 0.04 for at least some portion of the fermentation. More specifically, carbon dioxide levels are maintained below a partial pressure of about 0.04 prior to harvesting of the algal biomass for a sufficient time to increase carotenoid levels to the desired level.

The following experimental results are provided for purposes of illustration and are not intended to limit the scope of the invention.

Preparation of Inoculum and Medium

The following procedures were followed for Examples 1-6 and 8, as appropriate.

An inoculum was prepared using a strain of Neospongiococcum excentricum identified as ATCC No 40335. Initially, a Yeast-extract/Peptone (2X YEP) inoculum medium was prepared comprising 6.0 g/l of Difco~yeast extract, and 10.0 g/l of Bacto~peptone in 960 ml deionized water. The mixture was sterilized by autoclaving at 121°C for 25 minutes. Subsequent to autoclaving, 40 ml of a 50% glucose solution was added. A single colony of Neospongiococcum was inoculated into a 1 liter shaker flask containing 200 ml of the above-described inoculum medium. The flask was incubated in a qyrotory shaker at 400 rpm and at 35°C plus or minus 1°C. The flask was incubated until an optical density of 7 to 10 (absorbance at 620 nm) was attained, indicating mid-exponential growth phase.

A fermentation medium was prepared which includes 3.88 g/l of $KH_2PO_4$, 1.50 g/l of Amberex⁻ (Registered Trade Mark) yeast extract, 2.75 g/l of $(NH_4)_2SO_4$, and 0.20 g/l of citric acid in 9 1 of deionized water. This solution was autoclaved for 60 minutes at 121°C. Subsequently, the autoclaved solution was brought to 5 g/l of glucose, 5.0 ml/l of trace metal 8 solution, 4.5 ml/l of $MgSO_4 \cdot 7H_2O$, and 0.1 g/l of $FeSO_4 \cdot 7H_2O$.

Trace metal 8 solution includes, in 6.0 liters of deionized water, 200 ml of concentrated hydrochloric acid (97%), 114 grams of $CaCl \cdot 2H_2O$, 12.3 grams of manganese sulfate, 0.314 grams of copper sulfate·$5H_2O$, 1.6 grams of cobalt chloride·$6H_2O$, 9.12 grams of boric acid, 17.65 grams of zinc sulfate·$7H_2O$, 0.48 grams of sodium molybde-nate·$2H_2O$, 0.8 grams of vanadyl sulfate·$2H_2O$, 0.4 grams of nickel nitrate·$6H_2O$, 0.4 grams of sodium selenite, and 2.5 grams of sodium citrate. After all solids were added and fully dissolved, the final volume was brought to 10 liters with deionized water.

The pH of the fermentation medium was approximately pH 4.8. Ammonium hydroxide solution (14%) was used to adjust the pH to 6.0 prior to inoculation. Pre-inoculation conditions were as follows: temperature = 36 C ± 1 C; pH = 6.0 ± 0.2; agitation = 400 rpm; aeration = 1 vvm; and dissolved oxygen = 100% saturation.

The inoculum was aseptically transferred to the fermentation vessel. The final concentration of inoculum in the vessel was 5% by volume. The initial optical density was about 0.5.

For the fed-batch phase, the glucose fresh fermentation medium feed includes 500 g/l of glucose, which was autoclaved for 40 minutes at 121 C, with the subsequent addition of 28.57 ml/l of $MgSO_4 \cdot 7H_2O$ and 5.72 ml/l of $H_3PO_4$.

The continuous phase fresh fermentation medium feed stream includes sterilized glucose and 0.048 grams of $KH_2PO_4$ per gram of glucose, 0.0025 grams of $FeSO_4 \cdot 7H_2O$ per gram of glucose, 0.003 grams of anhydrous citric acid per gram of glucose, 0.11 ml of one molar $MgSO_4 \cdot 7H_2O$ per gram of glucose, and 0.125 ml of trace metal 8 solution per gram of glucose.

Example 1

A batch fermentation was conducted to evaluate the effects of temperature on heterotrophic algal growth and carotenoid formation. The fermentation was conducted using the strain of Neospongiococcum excentricum identified by ATCC No. 40335 in a Braun Biostat E fermenter. The fermentation was run for 35 hours. The pH of the medium was maintained at about pH 6.0. The dissolved oxygen content of the fermentation medium was maintained at greater than 20%. The inoculation was conducted and the fermentation medium was prepared as described above.

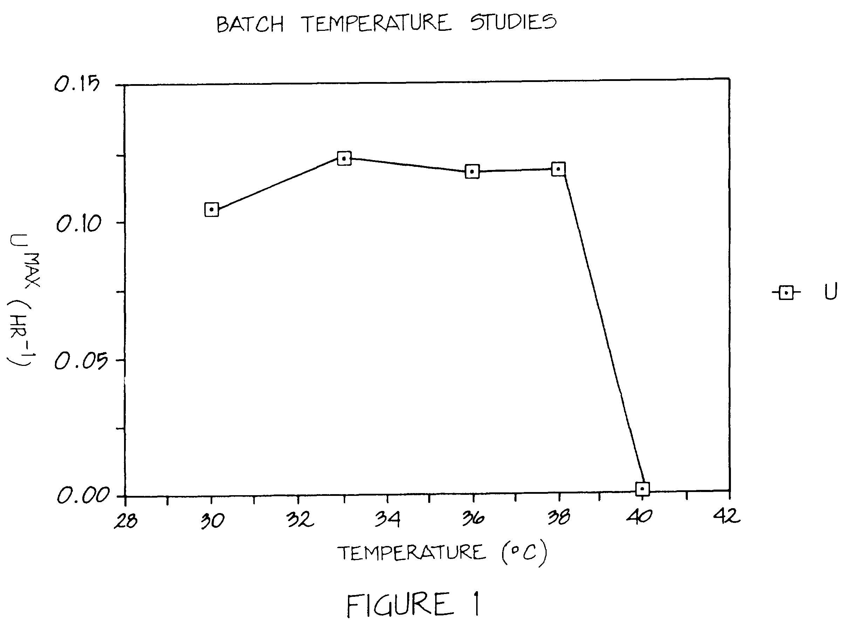

An inoculum of actively growing microorganisms was introduced into the fermentation medium. During the course of the fermentations, results were evaluated over the temperature range of 30°C to 40°C. The growth rate maxima ($u^{max}$) were determined by obtaining the slope of a best-fit line, on a semi-logarithmic plot, drawn through dry weight points taken over time for each temperature. The results of these fermentations given as average growth rates at the selected temperatures, are shown in Figure 1.

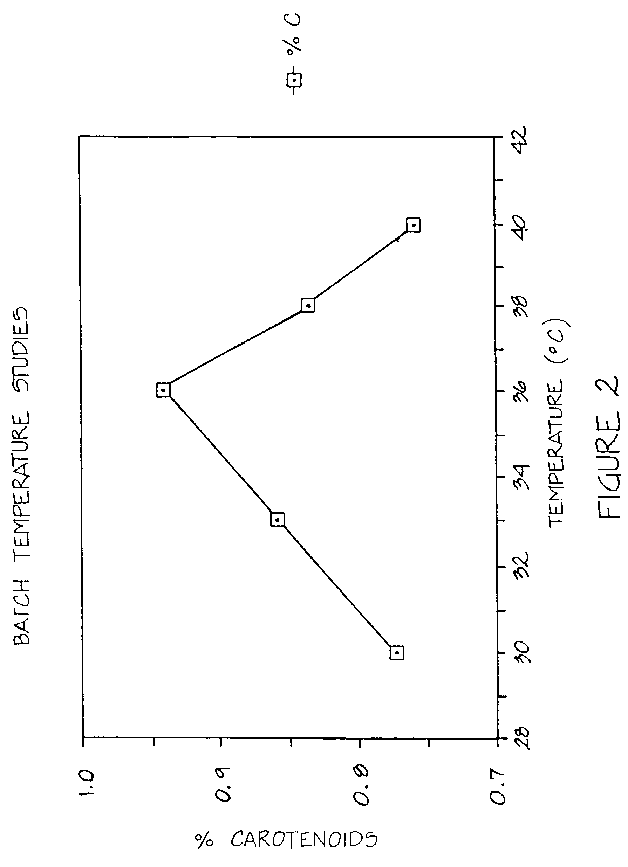

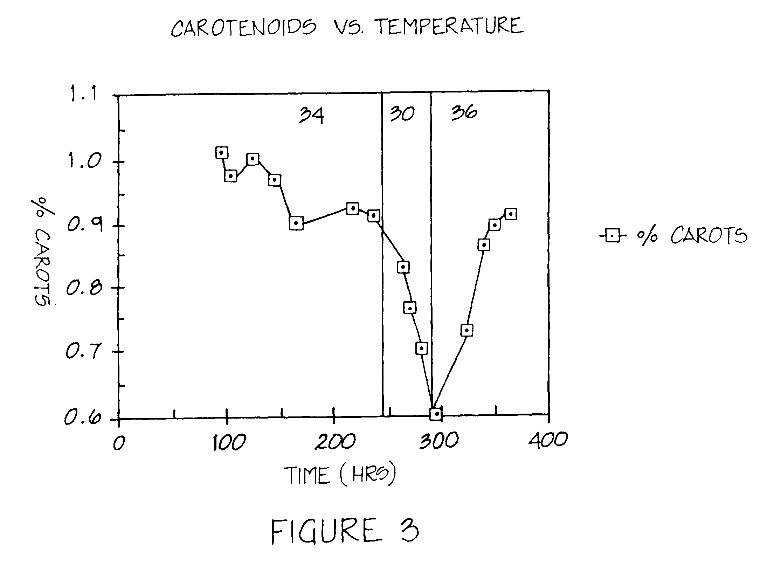

The percent of carotenoids per cell on a dry weight basis was also determined by averaging data from the fermentations taken at intermediate temperatures. These results are shown in Figure 2.

It can be seen from Figure 1 that high rates of growth are obtained at temperatures between about 33°C and about 38°C. Incubation at 40°C resulted in cell death. It should be noted, however, that these fermentations only study temperatures above 30°C and, therefore, effective fermentations may be conducted at temperatures lower than 30°C.

From Figure 2, it can be seen that carotenoid formation is highly dependent on temperature and is maximized between about 34°C and about 38°C, and is particularly maximized at about 36°C.

Example 2

The effect of temperature on carotenoid formation was also studied using data from chemostat fermentations. The strain of Neospongiccoccum excentricum studied was identified by ATCC No. 40335. The pH of the fermentation medium was maintained at about pH 6.0. The dissolved oxygen content of the fermentation medium was maintained at greater than 20%. The medium was prepared as described above.

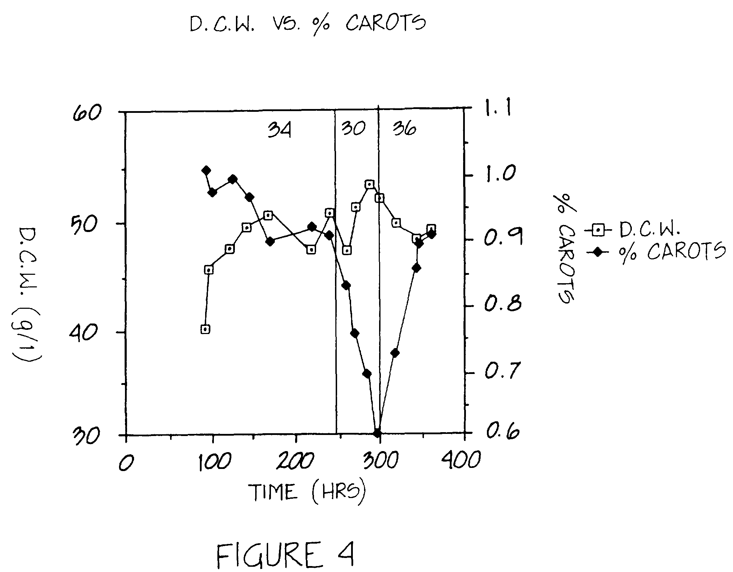

The tests were designed to illustrate the effect of shifting the temperature from 34°C to 30°C to 36°C. The temperatures are indicated at the top of Figs. 3 and 4. The results of the chemostat fermentation are shown in Figure 3. The effects of temperature on dry cell weight were also studied. The results, superimposed on Fig. 3, are shown in Figure 4.

As can be seen from Figure 4, a temperature of about 36°C provides for optimal carotenoid formation, when multiplied times the dry cell weight. Following a shift in temperature during steady state, maximum impact on carotenoid formation occurred three generations removed from the point of perturbation. These temperature-induced effects, however, are reversible within three generations upon return to the pre-perturbation steady state.

Example 3

The influence of pH on growth of <u>Neospongiococcum excentricum</u> strain ATCC No. 40335 was determined, once again, in a series of batch fermentations. Temperature was maintained at 36°C and pH was the variable. The pH was controlled by addition of $NH_4OH$ (14%). Growth rate maxima were determined at various pH levels.

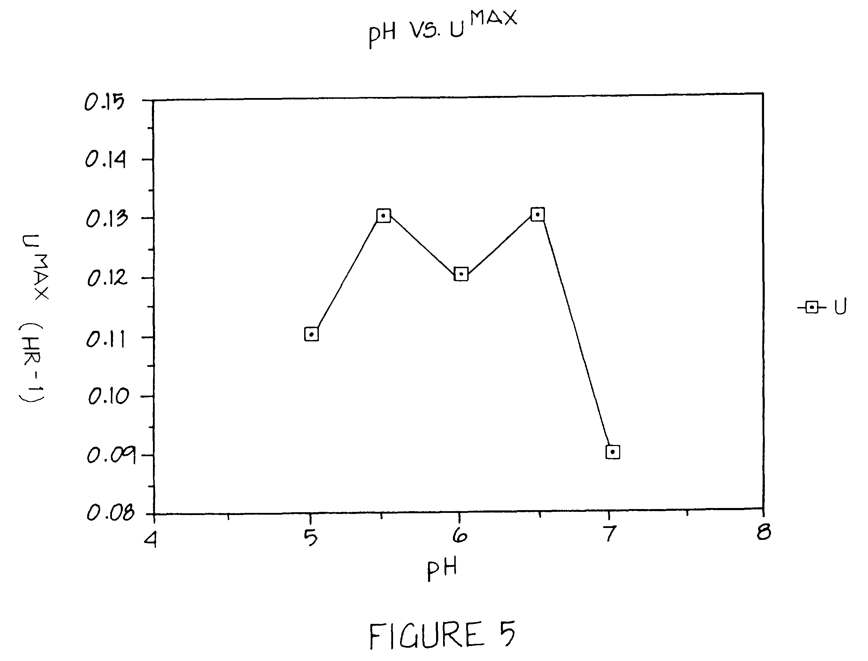

From these fermentations, a curve of pH vs. $u^{max}$ was plotted, as illustrated in Fig. 5. Based on these data, the effects of pH on growth indicates that a growth rate maximum at 36°C of 0.130 was achieved between pH 5.5 and 6.5, and that growth rate declined abruptly either below pH 5.5 or above pH 6.5, but more drastically above pH 6.5.

The effect of pH on carotenoid formation was determined by 2 methods: (1) averaging carotenoid values taken during a series of batch fermentations, and (2) shifting pH in glucose-limited steady state chemostats. Again, the latter method was employed to minimize any ambiguity caused by cell growth dynamics, and to arrive at a more predictable model of pH perturbations in the process.

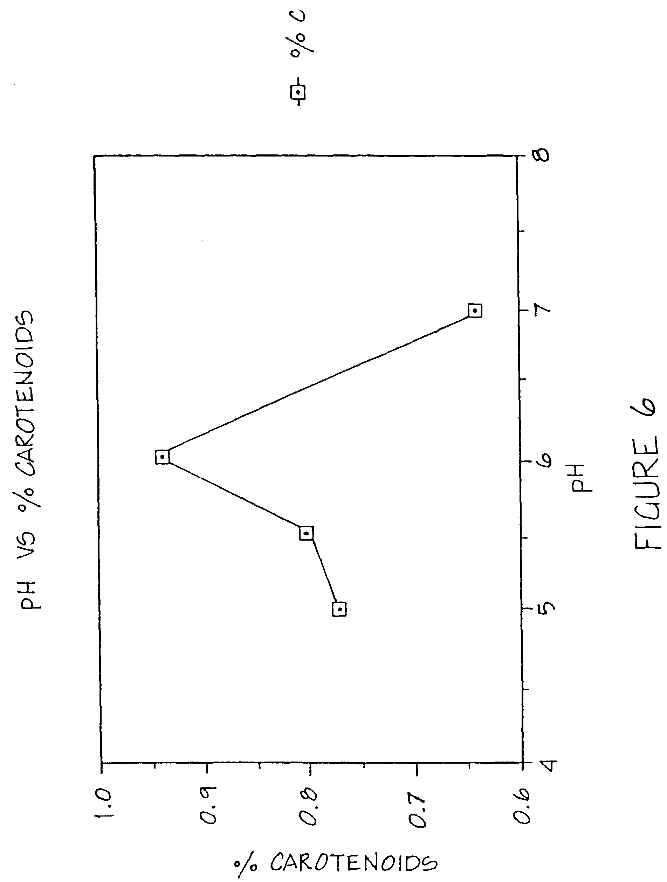

From the series of batch fermentations, a curve was obtained by plotting % carotenoids vs. pH. From this plot, shown in Fig. 6, it is clear that, maximum carotenoid formation at 36°C occurs at about pH 6.0, and that carotenoid formation is quite sensitive to pH, indicating the need for precise control of this parameter.

These results were also verified in a series of chemostat studies, whereby the pH set point was periodically adjusted either upward or downward. In these studies, as in the temperature studies, the maximum impact of pH perturbations required about 3 generations to appear. Also, as in the temperature studies, the effect on carotenoid formation was reversible within 3 generations once conditions were returned to normal.

Based on the above collective data, a pH set point optimum of about pH 6.0 is preferred, with a slight bias towards the lower end. Although the cells grow at near maximal growth rates between about pH 5.5 and about pH 6.5, it is the apparent sensitivity of the carotenogenic machinery in the cells which mandates precise control of pH.

Example 4

The effects of dissolved oxygen on the productivity of <u>Neospongiococcum</u> <u>excentricum</u> were assessed in steady-state chemostats (glucose-limited). Dissolved oxygen levels were maintained and adjusted by varying the agitation rate (rpm) while keeping the airflow constant at 1 volume of air per chemostat volume per minute.

In an initial study, a 10% Cerelose~ (trademark for refined glucose) feed was used with the dilution rate (D) at 0.03 to 0.04 per hour. The experimental dissolved oxygen levels tested included 70%, 20% and 10% of saturation. Cells were maintained at each level of dissolved oxygen for at least 4 generations. These data are summarized in Table 4.1. Based on these data, volumetric productivity as well as % carotenoids remained stable when dissolved oxygen was decreased from 70% saturation to 20% and finally 10% saturation. Productivity is equal to (gX/l) (mg cartenoids/g cells) (D, $hr^{-1}$) (0.72), where 0.72 is the factor relating carotenoid assay values to external Association of Official Analytical Chemistry (AOAC) xanthophyll values. The volumetric productivity figures are the steady state figures obtained during the continuous phase of the chemostat process.

Table 4.1
10% Glucose Feed

| % Dissolved $O_2$ | Xanthophyll Avg. % Carot. | Volumetric Productivity (mg/1/hr) | Avg. Yield Coefficient ($Y_{x/s}$) | Dilution Rate | Avg. $O_2$ Trans. Rate (millimols/1/hr) | Avg. Respiratory Quotient |
|---|---|---|---|---|---|---|
| 70 | 0.86 | 11.5 | 0.38 | 3.4% | 45.5 | 0.92 |
| 20 | 0.91 | 11.2 | 0.35 | 3.4% | 42.9 | 0.87 |
| 10 | 0.93 | 11.1 | 0.34 | 3.4% | 44.5 | 0.81 |

A second study was performed to further evaluate the response of cells to low-end dissolved oxygen levels (i.e. 10-20%) with a 13% Cerelose~ feed and near-maximal dilution rate (D=0.095 hr$^{-1}$). The test results are shown in Table 4.2. Under these conditions, there was no significant difference in productivity when the process was performed at 20% dissolved oxygen vs. 10% dissolved oxygen.

8

Table 4.2

| Effects of Dissolved Oxygen on Productivity | | | |
|---|---|---|---|
| Dilution Rate (hr$^{-1}$) | Dissolved Oxygen | Carot. Content | Xanthophyll Productivity (mg/l/hr) |
| 0.095 | 20% | 0.71% | 30 |
| 0.095 | 10% | 0.75% | 31 |

In spite of the demonstrated ability to successfully perform the process at low (10%) dissolved oxygen levels, it has been observed that oxygen limitation not only results in a loss of steady-state with glucose accumulation, but also has a dramatic negative effect on carotenoid formation. In these cases, a drastic decline in carotenoids occurs in less than a single generation, and is reversible within one generation, suggesting a physiological response.

Based on the above results, it appears that the continuous portion of the process may be performed at 10% dissolved oxygen. This parameter, of course, would be highly dependent on vessel configuration and mixing patterns. As the detrimental effects of oxygen limitation are well documented, care should be taken to maintain dissolved oxygen above this critical level.

The oxygen uptake rate (OUR) and respiratory quotients (RQ) were determined by mass-spectral analysis of the off-gas streams from steady-state chemostats run at various cell concentrations and flow rates. Typical OUR and RQ values obtained during one study in which the temperature was 36 C, the dissolved oxygen level was 20%, and a 13 weight/volume % Cerelose~ feed was added, are cited below on Table 4.3.

Table 4.3

| Dilution Rate | Dry cell weight (g/l) | OUR (mmol/1/hr) | RQ |
|---|---|---|---|
| 0.10 hr$^{-1}$ | 43.2 | 110 | 1.05 |

Example 5

A series of chemostat experiments were performed in order to assess the potentially toxic effect of carbon dioxide ($CO_2$) on cell growth and/or carotenoid formation. These experiments were performed by maintaining dissolved oxygen at about 20% and keeping airflow at a constant 1 volume air per chemostat volume per minute (vvm). Carbon dioxide gas was blended with incoming air to obtain the desired levels in the fermenter off-gas.

An initial chemostat study demonstrated the effects of gradually increasing the $CO_2$ in the off-gas from 2% to 10%. The results indicated that $CO_2$ levels exceeding 4% are detrimental to carotenoid formation. Increasing the $CO_2$ in the airstream from 4% to 10% resulted in a decline in carotenoids from 0.89% to 0.72%. On average, carotenoids declined in 0.03% increments for each 1% $CO_2$ in the off-gas. A significant increase in cell yield factor on glucose ($Y_{x/s}$, g cells/g glucose) was observed (0.32 vs. 0.35) at 10% $CO_2$. These results also demonstrated that, while elevated $CO_2$ levels inhibit carotenogenesis, there was no apparent adverse effect on cell growth.

A second chemostat study was performed in order to determine whether detrimental effects of $CO_2$ could be reversed once incurred. For this experiment, steady state cells at ambient (i.e. not $CO_2$ enriched) airflow were subjected to a 10% $CO_2$ environment for several generations, and then returned to ambient conditions.

Following $CO_2$ enrichment, carotenoid levels declined from 0.93% to 0.71% within one generation. When $CO_2$ levels were returned to ambient, carotenoid levels returned to near normal (about 0.90%) in less than one generation, indicating that the deleterious effects of $CO_2$, incurred during fermentation or holding of biomass, may be reversed by increasing ventilation (airflow) to the system. An extrapolation of these results may also be applied to determine a parameter for airflow. In tests, using an airflow of 1 vvm in the continuous phase with 13% (w/v) Cerelose~ and D=0.10 hr$^{-1}$, off-gas $CO_2$ averages about 4.5%. Therefore, while this airflow seems to pose minimal losses in productivity, additional ventilation should be considered if cell densities (i.e Cerelose~ feed concentration) is further increased.

Example 6

The following experimental results illustrate the productivity decline and cessation of growth of a microorganism fermentation in the absence of a continuous dilution of fermentation medium and restriction of a carbon source feed, as is disclosed in the present invention.

A fermentation was conducted using Neospongiococcum excentricum identified by ATCC No. 40335. The fermentation was conducted at a pH of about 6.0 and with a dissolved oxygen content of about greater than 2%.

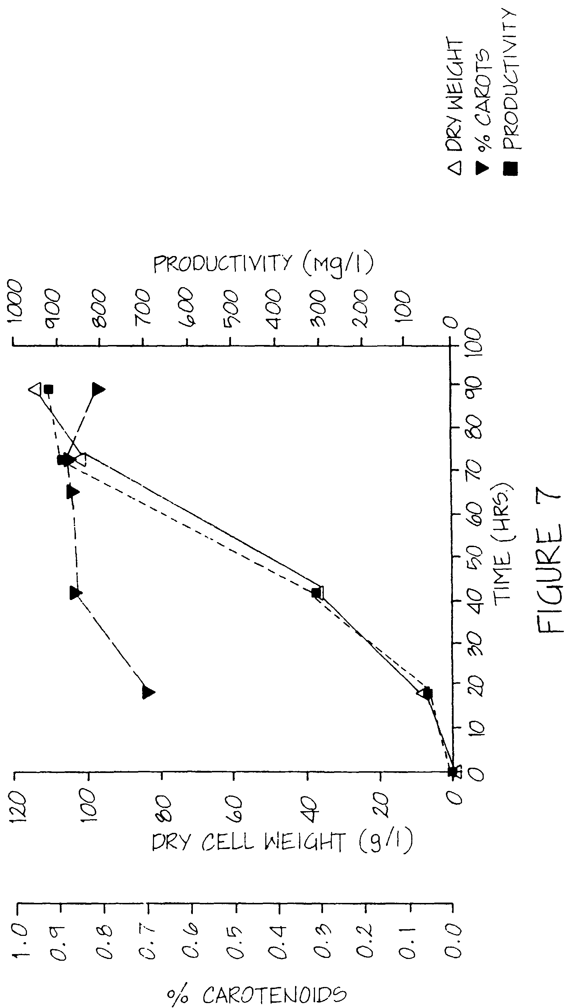

The results of this fermentation are shown in Figure 7. As can be seen from Figure 7, a cell density of about 113-114 g/l dry cell weight was obtained after 90 hours with 0.81% of the cell dry weight as carotenoids. The carotenoid productivity and cell growth ceased after 90 hours.

Example 7

The following example illustrates the use of the process parameters described above to achieve maximum productivity in a pilot plant fermentation.

A. Preparation of inoculum: A 14 liter fermenter was charged with 7 liters water containing 31 g KH$_2$PO$_4$, 30 g Amberex 695~ yeast extract, 22 g (NH$_4$)$_2$SO$_4$, and 1.0 ml of MAZU 204~ antifoam. The entire contents of the vessel were steam sterilized at 121°C for 60 min. A 500 ml solution of the remaining nutrients were then aseptically filter-sterilized into the fermenter. This nutrient solution contained 160 g glucose, 40 ml of trace metal ε solution, 9 g MgSO$_4$·7H$_2$O, 0.8 g FeSO$_4$·7H$_2$O, and 1.6 g citric acid. This fermenter is then inoculated with 500 ml of a 48 hr culture grown in YEP (9 g/l yeast extract, 15 g/l peptone, and 10 g/l glucose). Aeration is kept at 1.0-1.2 vvm with the agitation at 400-600 rpm throughout the run. The pH was controlled at 6.0 with anhydrous ammonia addition.

When the residual glucose decreases to approximately 5 g/l, a nutrient feed was initiated consisting of the following components:

| Glucose | 320 g/l |
|---|---|
| KH$_2$PO$_4$ | 62 g/l |
| (NH$_4$)SO$_4$ | 44 g/l |
| Trace metal 8 | 80 ml/l |
| MgSO$_4$·7H$_2$O | 17.8 g/l |
| FeSO$_4$·7H$_2$O | 1.6 g/l |
| Citric acid | 3.2 g/l |

This feed solution was maintained in such a fashion that the residual glucose in the fermenter ranges from 2-5 g/l. After approximately 24 hr of feed, the biomass accumulated to 40-50 g/l, and was used to inoculate the pilot fermenter.

B. Medium preparation and sterilization: A 450 liter fermenter was filled to 190 liter with water containing Sigma~ corn steep liquor (600 g -- 3.0 g/l) and MAZU 204~ antifoam (10 ml -- 0.05 ml/l), this solution was sterilized for 60 minutes at 121°C, and then cooled to 36°C. The following components were then added to 5 liters (final volume) of water, and the total contents aseptically added to the fermenter through a Millidisk sterile filter.

| Medium Component | Total Added | Final Concentration |
|---|---|---|
| Potassium Hydroxide (87.5%) | 348 g | 1.83 g/l |
| Phosphoric Acid (85%) | 625 g | 3.29 g/l |
| (NH$_4$)$_2$SO$_4$ | 523 g | 2.75 g/l |
| MgSO$_4$·7H$_2$O | 211 g | 1.11 g/l |
| Trace metal 8 solution | 950 ml | 5.0 ml/l |

The volume of the fermenter was adjusted to 200 liters with sterile water, and the pH adjusted to pH 6.0 with anhydrous ammonia.

C. Fed-batch fermentation phase: A this point, the entire contents of the 14 liter inoculum vessel was aseptically transferred to the 450 liter fermenter. When the residual glucose level in the fermenter decreased to 5 g/l (in approximately 12-15 hr), nutrient addition to the fermenter commenced. Two sterile nutrient feed streams were aseptically added to the fermenter in a 1:1 ratio such that the residual glucose was maintained at 3.5 g/l during this "fed-batch" portion of the fermentation. The two nutrient feed streams are listed below.

| Feed Solution #1 | Feed Solution #2 |
|---|---|
| Glucose (600 g/l) | Phosphoric acid (55.05 g/l) |
| FeSO$_4$·7H$_2$O (0.6 g/l) | Potassium hydroxide (29.8 g/l) |
| Citric acid (1.2 g/l) | Trace metal 8 solution (85.8 ml/l) |
|  | NH$_4$NO$_3$ (37.5 g/l) |

During this period of nutrient feeding, the pH was controlled at pH 6.0 ± 0.2 by the addition of anhydrous ammonia, and the dissolved oxygen was maintained at greater than 40% of saturation. After approximately 15 hr of nutrient feed, the biomass accumulated to 25-30 g/l, and the continuous phase of carotenoid production was initiated.

D. Continuous fermentation phase: After the biomass accumulated to approximately 25 g/l, the continuous phase of the fermentation was initiated. The continuous phase is characterized by allowing the glucose addition rate to become growth limiting, and by maintaining a fixed dilution rate once the desired dilution rate is achieved. In the present example, the target dilution rate was 0.08 hr$^{-1}$.

As in the fed-batch portion of the fermentation, the nutrient feed consists of two different sterile feed streams, and the composition of these streams is shown below. In this case, however, the ratio of the glucose feed to the salts feed is maintained at 1:3.

| Feed Solution #1 | Feed Solution #2 |
|---|---|
| Cerelose~ (600 g/l)<br>$FeSO_4 \cdot 7H_2O$ (0.6 g/l)<br>Citric acid (12 g/l) | Phosphoric acid (6.1 g/l)<br>KOH (34 g/l)<br>$MgSO_4 \cdot 7H_2O$ (47 g/l)<br>Trace metal 8 solution (4.8 ml/l)<br>$NH_4NO_3$ (4.2 g/l) |

In the present example, the combined nutrient feed was initiated to give an initial dilution rate of 0.06 hr$^{-1}$, and this dilution rate was increased to 0.07 hr$^{-1}$ and finally 0.08 hr$^{-1}$ in 10 hr increments. The dilution rate was then maintained at this level for approximately 80 hr of continuous operation. During this continuous fermentation phase, the carotenoid level of the biomass averaged 0.85% (this calculates to 0.61% AOAC xanthophylls using the 0.72 correlation factor). The cumulative volumetric productivity for the run described was 24.7 mg AOAC xanthophyll/l-hr.

Example 8

The following results illustrate the increase in cell yield per unit of glucose by limiting glucose in a fermentation medium. In a continuous growth phase of a fermentation using a strain of Neospongiococcum excentricum identified by ATCC No. 40335, as described above, the dilution rate was increased from 0.073 to 0.139 hr$^{-1}$ and the corresponding volumetric productivity was measured. The results of this investigation are shown in Table 8.1.

Table 8.1

| Effect of Dilution Rate on Steady State Growth and Carotenoid Yields in a Glucose-Limited Chemostat | | | | |
|---|---|---|---|---|
| Dilution Rate,hr$^{-1}$ | Biomass g/l | Cell Yield $Y_{x/s}$ | Carotenoids % | Xanthophyll Volumetric Productivity mg / 1 / hr |
| 0.073 | 9.0 | 0.40 | 0.98 | 4.6 |
| 0.096 | 10.1 | 0.41 | 0.97 | 6.8 |
| 0.11 | 11.3 | 0.44 | 0.85 | 7.6 |
| 0.139[1] | 4.8 | -- | 0.70 | 3.4 |

[1] Wash out started - Values may not reflect true steady state.

Example 9

In order to demonstrate the improved productivities obtained with the present invention, calculations are made in order to determine the volumetric productivity of prior art processes and the present processes. For Example 9 only, the volumetric productivity was calculated on a cumulative basis by multiplying the amount of biomass (in grams per liter) times the carotenoid content (in milligrams carotenoids per gram of cells) times 0.72 (where 0.72 is the factor relating carotenoid assay values to external xanthophyll values according to the Association of Official Analytical Chemists) and dividing by the total reaction time (in hours). This was necessary to accurately compare the total productivity of prior art batch reactions to the present continuous method. In other words:

volumetric productivity = (g biomass/liter broth)(mg carotenoids/g cells)(0.72)/(hr reaction time)

The values obtained are shown in Table 9.1.

Table 9.1

| COMPARISON OF XANTHOPHYLL PRODUCTIVITY VALUES | | |
|---|---|---|
| Reference | Organism | Productivity (mg/l/hr) |
| US Patent #2,949,700 (Corn Processing Corp) | Chlorella sp | 0.30 |
| US Patent #3,280,502 (Hoffmann-La Roche) | Chlorella sp | 2.5 |
| Theriault, 1965 | Chlorella sp | 4.0 |
| A. Ciegler (1956 Adv. Appl. Microbiol. 7:1-34) | Neospongiococcum | 1.36 |
| Pat. Appl. #07/058,512 (Coors BioTech, Inc.) | Neospongiococcum | 7.0 |
| Present Process (Coors BioTech, Inc.) | Neospongiococcum | greater than 10 |

Table 9.1 indicates the productivity gains resulting form the method of the present invention.

While various embodiments of the present invention have been described in detail, it is apparent that modifications and adaptations of those embodiments will occur to those skilled in the art. However, it is to be expressly understood that such modifications and adaptations are within the scope of the present invention, as set forth in the following claims.

## Claims

1. A microorganism having all the identifying characteristics of Neospongiococcum excentricum ATCC 40335 or a mutant thereof which is capable of producing xanthophyll at a rate of at least 10mg/l/hr.

2. A method for producing carotenoids by continuous fermentation of a microorganism of the species Neospongiococcum excentricum which has all the identifying characteristics of Neospongiococcum excentricum ATCC No 40335, or a mutant thereof capable of producing xanthophyll at a rate of at least 10 mg/l/hr comprising:

   (a) continuously adding fresh fermentation medium comprising a nutrient to a fermentation broth comprising said microorganism, wherein said nutrient is selected from the group consisting of carbon, nitrogen, phosphate, sulfate, magnesium, and iron, and, wherein the rate of addition of said nutrient is less than that necessary to provide for a maximum growth rate of said microorganism;
   (b) maintaining the pH of said fermentation broth between pH 5.5 and pH 6.5; and
   (c) maintaining the temperature of said fermentation broth between 30°C and 38°C.

3. A method, as claimed in Claim 2, wherein said microorganism produces xanthophyll at a rate of at least 10 mg/l/hr.

4. A method, as claimed in Claim 2, wherein said microorganism produces xanthophyll at a rate of at least 15 mg/l/hr.

5. A method, as claimed in Claim 2, wherein said microorganism produces xanthophyll at a rate of at least 20 mg/l/hr.

6. A method, as claimed in Claim 2, wherein said nutrient is a carbon source.

7. A method, as claimed in Claim 2, wherein said nutrient is glucose or sucrose.

8. A method, as claimed in Claim 2, wherein said rate of addition is from 0.03 hr$^{-1}$ to 0.13 hr$^{-1}$.

9. A method, as claimed in Claim 2, further comprising removing spent fermentation broth, wherein the volume of said fermentation broth is maintained substantially constant by maintaining substantially equal in-flow of fresh fermentation medium and out-flow of spent fermentation broth.

10. A method, as claimed in Claim 2, wherein the pH of said fermentation broth is pH 6.0.

# EP 0 553 085 B1

11. A method, as claimed in Claim 2, wherein the temperature of said fermentation broth is between 33°C and 38°C.

12. A method, as claimed in Claim 2, wherein the temperature is maintained at 36°C.

13. A method, as claimed in Claim 2, wherein the growth rate of said microorganisms is from 25% to 95% of the maximum growth rate.

14. A method, as claimed in Claim 2, wherein said fermentation broth contains greater than 10% dissolved oxygen.

15. A method, as claimed in Claim 2, wherein the partial pressure of carbon dioxide in said fermentation broth is less than 0.04 atmospheres.


**Patentansprüche**

1. Ein Mikroorganismus mit allen kennzeichnenden Merkmalen von Neospongiococcum excentricum ATCC 40335 oder einer Mutante davon, und fähig ist, Xantophyll mit einer Rate von wenigstens 10 mg/l/h herzustellen.

2. Ein Verfahren zur Herstellung von Carotinoiden durch kontinuierliche Fermentation eines Mikroorganismus der Art Neospongiococcum excentricum, der alle kennzeichnenden Merkmale von Neospongiococcum excentricum ATCC 40335 oder einer Mutante davon aufweist, und fähig ist, Xantophyll mit einer Rate von wenigstens 10 mg/l/h herzustellen, wobei die folgenden Schritte umfaßt sind:

   (a) kontinuierliches Zugeben von frischem Fermentationsmedium, das einen Nährstoff aufweist, zu einer Fermentationsbrühe, welche die genannten Mikroorganismen aufweist, wobei der genannte Nährstoff aus der Gruppe gewählt ist, die aus Kohlenstoff, Stickstoff, Phosphat, Sulfat, Magnesium und Eisen besteht, und wobei die Zugaberate des genannten Nährstoffes kleiner ist als jene, die notwendig ist, um für eine maximale Wachstumsrate des genannten Mikroorganismus zu sorgen;
   (b) Halten des pH der genannten Fermentationsbrühe zwischen pH 5,5 und pH 6,5; und
   (c) Halten der Temperatur der genannten Fermentationsbrühe zwischen 30°C und 38°C.

3. Ein Verfahren nach Anspruch 2, bei welchem der genannte Mikroorganismus Xantophyll mit einer Rate von wenigstens 10 mg/l/h herstellt.

4. Ein Verfahren nach Anspruch 2, bei welchem der genannte Mikroorganismus Xantophyll mit einer Rate von wenigstens 15 mg/l/h herstellt.

5. Ein Verfahren nach Anspruch 2, bei welchem der genannte Mikroorganismus Xantophyll mit einer Rate von wenigstens 20 mg/l/h herstellt.

6. Ein Verfahren nach Anspruch 2, bei welchem der genannte Nährstoff eine Kohlenstoffquelle ist.

7. Ein Verfahren nach Anspruch 2, bei welchem der genannte Nährstoff Glucose oder Saccharose ist.

8. Ein Verfahren nach Anspruch 2, bei welchem die genannte Zugaberate zwischen 0,03 h$^{-1}$ und 0,13 h$^{-1}$ liegt.

9. Ein Verfahren nach Anspruch 2, welches weiterhin das Entfernen von verbrauchter Fermentationsbrühe umfaßt, wobei das Volumen der genannten Fermentationsbrühe im wesentlichen konstant gehalten wird, indem der Zulauf von frischem Fermentationsmedium und der Ablauf von verbrauchter Fermentationsbrühe im wesentlichen gleich gehalten wird.

10. Ein Verfahren nach Anspruch 2, bei welchem der pH-Wert der genannten Fermentationsbrühe pH 6,0 ist.

11. Ein Verfahren nach Anspruch 2, bei welchem die Temperatur der genannten Fermentationsbrühe zwischen 33°C und 38°C liegt.

12. Ein Verfahren nach Anspruch 2, bei welchem die Temperatur auf 36°C gehalten wird.

**13.** Ein Verfahren nach Anspruch 2, bei welchem die Wachstumsrate der genannten Mikroorganismen 25 % bis 95 % der maximalen Wachstumsrate beträgt.

**14.** Ein Verfahren nach Anspruch 2, bei welchem die genannte Fermentationsbrühe mehr als 10 % gelösten Sauerstoff enthält.

**15.** Ein Verfahren nach Anspruch 2, bei welchem der Partialdruck von Kohlendioxid in genannter Fermentatiosbrühe weniger als 4 Atmosphären beträgt.

**Revendications**

**1.** Micro-organisme ayant toutes les caractéristiques d'identification du Neospongiococcum excentricum ATCC 40335, ou d'un mutant de celui-ci, qui est capable de produire de la xanthophylle à un taux d'au moins 10 mg/l/h.

**2.** Procédé de production de caroténoïdes par fermentation continue d'un micro-organisme de l'espèce Neospongiococcum excentricum qui a toutes les caractéristiques d'identification du Neospongiococcum excentricum ATCC No 40335, ou d'un mutant de celui-ci, capable de produire de la xanthophylle à un taux d'au moins 10 mg/l/h, comprenant:

(a) l'addition en continu d'un milieu de fermentation frais comprenant une substance nutritive à un bouillon de fermentation comprenant ledit micro-organisme, dans lequel ladite substance nutritive est sélectionnée dans le groupe consistant en carbone, azote, phosphate, sulfate, magnésium, et fer, et, dans lequel le taux d'addition de ladite substance nutritive est inférieur à celui qui est nécessaire pour fournir un taux de croissance maximum dudit micro-organisme ;
(b) le maintien du pH dudit bouillon de fermentation entre pH 5,5 et pH 6,5 ; et
(c) le maintien de la température dudit bouillon de fermentation entre 30 °C et 38 °C.

**3.** Procédé selon la revendication 2, dans lequel ledit micro-organisme produit de la xantrophylle à un taux d'au moins 10 mg/l/h.

**4.** Procédé selon la revendication 2, dans lequel ledit micro-organisme produit de la xantrophylle à un taux d'au moins 15 mg/l/h.

**5.** Procédé selon la revendication 2, dans lequel ledit micro-organisme produit de la xantrophylle à un taux d'au moins 20 mg/l/h.

**6.** Procédé selon la revendication 2, dans lequel ladite substance nutritive est une source de carbone.

**7.** Procédé selon la revendication 2, dans lequel ladite substance nutritive est du glucose ou du saccharose.

**8.** Procédé selon la revendication 2, dans lequel ledit taux d'addition est compris entre 0,03 $h^{-1}$ et 0,13 $h^{-1}$.

**9.** Procédé selon la revendication 2, comprenant, de plus, l'élimination du bouillon de fermentation utilisé, dans lequel le volume dudit bouillon de fermentation est maintenu sensiblement constant en maintenant sensiblement égal le débit d'entrée du milieu de fermentation frais et le débit de sortie du bouillon de fermentation utilisé.

**10.** Procédé selon la revendication 2, dans lequel le pH dudit bouillon de fermentation est de 6,0.

**11.** Procédé selon la revendication 2, dans lequel la température dudit bouillon de fermentation est comprise entre 33 °C et 38 °C.

**12.** Procédé selon la revendication 2, dans lequel la température est maintenue à 36 °C.

**13.** Procédé selon la revendication 2, dans lequel le taux de croissance desdits micro-organismes est compris entre 25 % et 95 % du taux de croissance maximum.

**14.** Procédé selon la revendication 2, dans lequel ledit bouillon de fermentation contient plus de 10 % d'oxygène dissous.

15. Procédé selon la revendication 2, dans lequel la pression partielle de gaz carbonique dans ledit bouillon de fermentation est inférieure à 0,04 atmosphère.

BATCH TEMPERATURE STUDIES

TEMPERATURE (°C)

$U^{MAX}$ (HR$^{-1}$)

FIGURE 1

BATCH TEMPERATURE STUDIES

FIGURE 2

CAROTENOIDS VS. TEMPERATURE

FIGURE 3

EP 0 553 085 B1

FIGURE 4

pH VS. U<sup>MAX</sup>

FIGURE 5

FIGURE 6

FIGURE 7

△ DRY WEIGHT
▼ % CAROTS
■ PRODUCTIVITY